# EUROPEAN PATENT SPECIFICATION

(11) **EP 1 781 203 B1**
(45) Date of publication and mention of the grant of the patent: **11.09.2019**
(21) Application number: 05761373.9
(22) Date of filing: 17.06.2005
(51) Int. Cl.: B29C 70/70

(54) **METHODS AND SYSTEMS FOR LOADING AN IMPLANTABLE MEDICAL DEVICE WITH BENEFICIAL AGENT**
VERFAHREN UND SYSTEME ZUM BELADEN EINES IMPLANTIERBAREN MEDIZINPRODUKTS MIT EINEM VORTEILHAFTEN MITTEL
PROCÉDÉS ET SYSTÈMES DE CHARGEMENT D'UN DISPOSITIF MÉDICAL IMPLANTABLE AVEC UN AGENT BÉNÉFIQUE

(30) Priority: 24.06.2004 US 876520
(43) Date of publication of application: 09.05.2007
(73) Proprietor: Shanghai MicroPort Medical (Group) Co., Ltd., Shanghai 201203 (CN)
(72) Inventor: PARKER, Theodore, L., Danville, CA 94506 (US); SHANLEY, John, F., Redwood City, CA 94061 (US)
(74) Representative: Diehl & Partner GbR
(86) International application number: PCT/US2005/021532
(87) International publication number: WO 2006/012060

(56) References cited:
- WO-A1-2004/043300
- US-A- 5 922 393
- US-A1- 2003 028 243
- US-A1- 2004 073 294

## Description

### Background

Implantable medical devices are often used for delivery of a therapeutic agent, such as a drug, to an organ or tissue in the body at a controlled delivery rate over an extended period of time. These devices may be able to deliver agents to different bodily systems to provide a variety of treatments.

One of the implantable medical devices which has been used for local delivery of therapeutic agents is the stent. Stents are typically introduced percutaneously, and transported transluminally until positioned at a desired location within a body lumen. These devices are then expanded either mechanically, such as by the expansion of a mandrel or balloon positioned inside the device, or expand themselves by releasing stored energy upon actuation within the body. Once expanded within a body lumen, the stent becomes encapsulated within the body tissue and remains a permanent implant.

Of the many problems that may be addressed through stent-based local delivery of therapeutic agents, one of the most important is restenosis. Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the vessel lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

One of the techniques under development to address the problem of restenosis is the use of surface coatings of various therapeutic agents on stents. U.S. Pat. No. 5,716,981, for example, discloses a stent that is surface-coated with a composition comprising a polymer carrier and paclitaxel (a well-known compound that is commonly used in the treatment of cancerous tumors). Known surface coatings, however, can provide little actual control over the release kinetics of therapeutic agents. These coatings are generally very thin, typically 5 to 8 microns deep. The surface area of the stent, by comparison is very large, so that the entire volume of the therapeutic agent has a very short diffusion path to discharge into the surrounding tissue.

In addition, it is not currently possible to deliver some drugs with a surface coating for a variety of reasons. In some cases, the drugs are sensitive to water, other compounds, or conditions in the body which degrade the drugs. Other drugs are not compatible with the polymer materials which function well as surface coatings.

U.S. Patent Publication No. 2004/0073294 describes a system for loading a beneficial agent into openings in an implantable medical device, such as a stent. The dispensing of a plurality of layers of one or more solutions into the openings is described to form a beneficial agent matrix for controlled release of a drug. The beneficial agent morphologies which can be formed within openings in a stent greatly increase the controllability of drug delivery and increase the variety of drugs that can be delivered. However, inconsistencies, such as holes, have been observed in some of the layers formed by these methods which can affect the matrix morphology and the consistency of the resulting drug delivery profile.

Accordingly, it would be desirable to provide a medical device with a system and method having improved control over beneficial agent matrix morphology by controlling the structure of the layers which form the matrix.

Document US 2003/0028243 A1 discloses coating an implantable medical device such as a coronary stent with a layer of a bioactive material and, posited over the bioactive material layer, a porous layer made by condensation of a polyamide or parylene monomer vapor, without solvents.

### Summary of the Invention

The present invention relates to methods and systems for loading an implantable medical device with a beneficial agent in which surface energies and/or surface tensions are controlled to achieve desired wetting characteristics. Control of these wetting characteristics is translated directly to control of the elution kinetics of a drug from the stent. The invention relates to a method as defined by claim 1 and a system as defined by claim 16.

In accordance with one aspect of the present invention, a method includes
providing an implantable device body having a plurality of through holes, substantially sealing a bottom of the plurality of holes with a temporary sealing member, the temporary sealing member having a surface energy, and loading a liquid beneficial agent into the holes. A surface tension of the liquid beneficial agent is less than the surface energy of the sealing member to achieve wetting of the temporary sealing member.

In accordance with another aspect of the invention, a system for loading an implantable medical device with a beneficial agent includes an implantable medical device having holes therein, a bottom surface of the holes formed of a different material than the implantable medical device, the bottom surface of the holes having a surface energy, a dispenser for dispensing a liquid solution of beneficial agent into the holes, and a supply of the liquid solution connected to the dispenser. The liquid solution has a surface tension less than the surface energy of the bottom surface of the holes.

### Brief Description of the Drawings

The invention will now be described in greater detail with reference to the preferred embodiments illustrated in the accompanying drawings, in which like elements bear like reference numerals, and wherein:
FIG. 1 is a side cross sectional view of a portion of a stent being loaded with a beneficial agent by a dispenser.
FIG. 2 is a photograph of a portion of a stent strut having holes showing an uneven layer in the bottom of the holes.
FIG. 3 is a photograph of a portion of a stent strut having holes showing an evenly layer in the bottom of the holes.

### Detailed Description

A method and system for loading an implantable medical device with a beneficial agent deposits a liquid solution into a plurality of holes in the medical device. The liquid solution is then solidified to form a first layer or portion of a beneficial agent matrix. The depositing and solidifying steps are repeated to form a controlled drug delivery matrix within the holes. The solidification process may be an evaporative process where a volatile component, often a solvent, is removed to leave a solid residue. The solidification process may also be a conversion process where the fluent liquid is converted into a non-fluent state such as by cross-linking or polymerization of component(s) of this fluent mixture.

Wetting characteristics within the holes have been found to affect the matrix formed within the holes. The selection and control of the surface energies of one or more of the surfaces within the holes and the surface tension of the filling solution allow the system to achieve desired wetting characteristics. The wetting characteristics control the uniformity of the layers within the holes and affect the morphology of the formed matrix. The morphology of the matrix affects the elution kinetics of a therapeutic agent delivered from the matrix.

### Definitions

The following terms, as used herein, shall have the following meanings:
The term "beneficial agent" as used herein is intended to have the broadest possible interpretation and is used to include any therapeutic agent or drug, as well as inactive agents such as barrier layers, carrier layers, base layers, cap layers, therapeutic layers, separating layers, or protective layers.

The terms "drug" and "therapeutic agent" are used interchangeably to refer to any therapeutically active substance that is delivered to a living being to produce a desired, usually beneficial, effect.

The terms "matrix" or "biocompatible matrix" are used interchangeably to refer to a medium or material that, upon implantation in a subject, does not elicit a detrimental response sufficient to result in the rejection of the matrix. The matrix may contain or surround a therapeutic agent, and/or modulate the release of the therapeutic agent into the body. A matrix is also a medium that may simply provide support, structural integrity or structural barriers. The matrix may be polymeric, non-polymeric (e.g. carbohydrates, disaccharides, oligosaccharides, polysaccharides and polysaccharide derivatives such as trehalose or sucrose), hydrophobic, hydrophilic, lipophilic, amphiphilic, mixtures thereof and the like. The matrix may be bioresorbable or non-bioresorbable.

The term "bioresorbable" refers to a matrix, as defined herein, that can be broken down by either chemical or physical process, upon interaction with a physiological environment. The matrix can erode or dissolve. A bioresorbable matrix serves a temporary function in the body, such as drug delivery, and is then degraded or broken into components that are metabolizable or excretable, over a period of time from minutes to years, preferably less than one year, while maintaining any requisite structural integrity in that same time period.

The term "openings" includes holes, through openings, grooves, channels, recesses, and the like.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a Mw greater than about 3000 and preferably greater than about 10,000 and a Mw that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); poly(vinylpyrrolidone) (PVP); polyorthoesters; disaccharides, oligosaccharides, polysaccharides and polysaccharide derivatives such as trehalose or sucrose, polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.

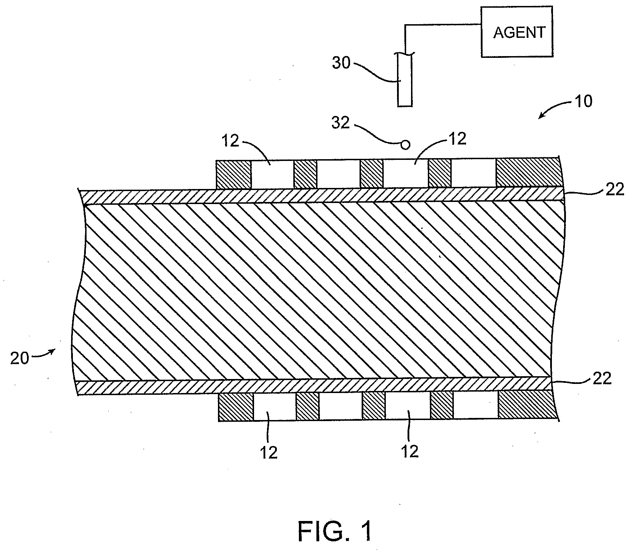

FIG. 1 is a cross section showing a portion of a medical device 10, such as a stent, positioned on a mandrel 20 for filling with a beneficial agent. The medical device 10 includes a plurality of holes 12 which are configured to be filled with the beneficial agent, such as a drug/polymer matrix for delivery in a controlled manner to a patient. A dispenser 30 is arranged to deposit a liquid solution into the holes 12 to form the drug/polymer matrix. Examples of dispensers 30 and loading systems are described in U.S. Patent Application Serial No. 10/668,125, filed on September 22, 2003, which is incorporated herein by reference in its entirety.

During the filling process, the medical device 10 is positioned on a mandrel 20 with a rubber-like surface material 22 which serves as a temporary bottom surface of the holes 12. Once the liquid material deposited within the holes 12 has been partially or completely solidified by removal of solvent or another solidification process, the medical device 10 can be removed from the mandrel 20.

In one example, the dispenser 30 is a piezoelectric dispenser which dispenses a plurality of drop 32 of a liquid solution into each of the holes 12 of the medical device 10. The liquid solution is deposited in a plurality of filling and drying steps to create a drug/polymer matrix. By variation of the amount and the composition of the drug and/or polymer dissolved or suspended in the fluent liquid solution deposited in each loading step, different drug/polymer matrix morphologies within the holes 12 can be achieved. The arrangement of drug and polymer in the matrix can be tailored to achieve a desired drug delivery profile with layers performing different functions. For example, base layers, barrier layers, cap layers, separating layers, or protective layers substantially without drug can be used in combination with drug layers to create specific drug delivery profiles. These methods can be used to control direction of drug delivery, rate of drug delivery, administration period, as well as control of the release profile and direction for more than one drug.

The volume of each hole 12 is low, on the order of about 0.1 nanoliters to about 50 nanoliters, preferably about 0.2 nanoliters (nl) to about 3 nl, and the size of the hole is small, on the order of about 50 - 300 micrometers (µm), preferably less than about 150 µm in diameter for a round hole by about 50 - 300 µm, preferably less than about 150 µm deep. With these small size holes, the behavior of a liquid solution within the holes is largely determined by the surface energies of the walls of the holes, the surface energy of the temporary bottom of the holes, and the surface tension of the liquid solution. Generally, the interaction of the liquid solution with the walls and bottom of the holes 12 can be described by the concept of wettability, that is, the ability of the liquid phase to advance over and cover the solid phase (walls and bottom). The behavior of the liquid solution on these surfaces controls the position and geometry of the solid polymer or polymer/drug mixture formed in the holes after solvent evaporation.

For stents made of stainless steel, cobalt chrome alloy, or other metals, the metal walls have a relatively high surface energy, while the temporary bottom 22, formed of silicone rubber or other resilient material, has a relatively low surface energy. The walls of the holes then are relatively easily wetted, whereas the silicone rubber temporary bottom is more difficult to wet.

Consequently, when the fluid solution is evaporated, often the resultant solid is deposited largely on the walls of the holes 12 and less on the bottom. In fact, in some cases, a portion of the bottom is not covered at all, leaving a hole, or creating a marked meniscus effect around the edges of the hole. When a minimum thickness of pure polymer solid is desired on the bottom of the hole to retard or control the release of the drug, such geometries severely limit the ability to deliver the drug in a controlled, sustained, or prolonged fashion.

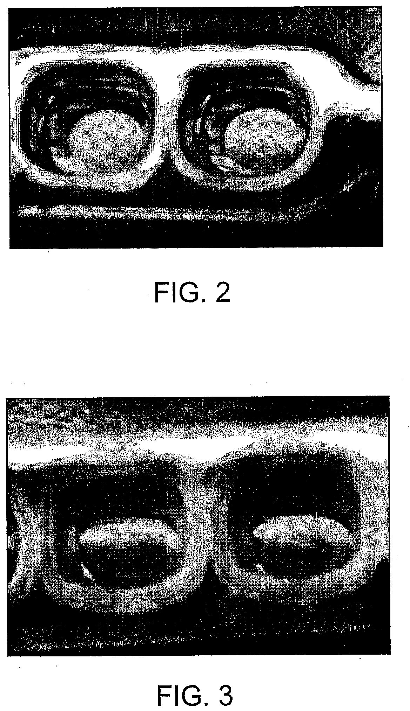

FIG. 2 is a photograph taken of a stent with holes loaded with a polymer material. As shown in the photograph, the polymer has incompletely covered the bottom leaving a hole in the polymer layer. In FIG. 2, the patterned patina of the surface of the mandrel can viewed in the bottom of the hole indicating that solidified polymer layer has not completely covered the bottom. A meniscus of polymer can be seen around the edge of the hole.

It has also been found that there can be a templating effect wherein the deposition geometry of the solid deposited in the first filling step tends to influence the geometry of subsequent material deposited on top of the first layer. Consequently, complete coverage of the temporary bottom of the hole with solid in the first process step is advantageous and leads to increased uniformity of subsequent layers.

The geometry of the solid deposited in the bottom of the hole can be controlled by controlling the relative surface energies surfaces of the surfaces of the hole in the stent and/or the surface tension of the liquid solution. By controlling the geometry of the solid in the stent holes, the release kinetics of the drug delivered from the stent to the tissue of a patient can be more precisely controlled.

In one example, the surface energy of the temporary bottom of the holes is controlled by selection of material or by treatment of the temporary bottom material by one of the treatment processes which will be described below. When the surface energy of the temporary sealing member at the bottom of the hole is selected to be higher than the surface tension of the liquid solution deposited in the holes wetting of the entire surface will occur and consistent coverage of the hole bottoms will be achieved. FIG. 3 is a photograph of a stent having a uniform layer of polymer material at the bottom without noticeable holes. In this case the mandrel surface on which the stent is mounted was treated to increase its surface energy resulting in the improved coverage.

In some instances the low surface energy of the silicone rubber temporary bottom will be increased from its natural state and the high surface energy of the metal walls will be reduced from its natural state. Such a modification of relative surface energies will allow the fluid solution to more fully fill the bottom of the holes, which after solvent evaporation will create a solid polymer or drug/polymer structure where the thickness of the solid layer is more consistent over the bottom of the hole with less meniscus at the walls than is achievable without modification of one or more of the respective surfaces. The surface energy of the walls if reduced significantly can cause difficulty in retaining the matrix in the holes.

The treatment of the temporary bottom surface of the holes to increase the wettability of the surface can involve extraction, oxidation, coating, and/or other treatment processes which increase the surface energy of a polymer or rubber-like material. The treatments described below are some of those processes useful for a silicone rubber surface, such as the surface of silicone tubing, particularly silicone tubing mounted on a mandrel whereon stents to be filled are crimped. However, similar treatments can be used for surfaces of other shapes and materials.

Generally, the method to increase the wettability of the silicone rubber surface can be a treatment which increases the number or density of polar groups on the surface, such as hydroxyl groups (-OH), carbonyl groups (>C=O), acid groups (-COOH), and the like. Further, the method can be an oxidation method, such as chemical oxidation with an oxidative reagent, flame oxidation, plasma oxidation (Argon with air or oxygen quench, oxygen, nitrous oxide plasmas), corona discharge oxidation and the like. Preferably, the treatment methods increase the surface energy of the mandrel by at least 2 dynes/cm. The treatment selected may differ depending on the liquid solution to be loaded since a desired surface energy of the mandrel is approximately equal to or greater than the surface tension of the liquid solution.

### Plasma Oxidation Methods

Plasma oxidation methods involve exposing at least a portion of a surface of a the mandrel to a chemically oxidizing plasma environment either in a one step or a two step process.

Chemically oxidizing environments include the following exemplary processes.
(1) One chemically oxidizing treatment includes vacuum or atmospheric plasma treatment with an inert process gas and an oxidizing process gas or oxidizing process aerosol. Inert process gasses include noble gasses, such as argon and helium, preferably argon. The process gas and/or process aerosol with an oxidizing action can be present in proportion of up to 100%, preferably between 5% and 95% by volume based on total volume of the process gas and/or process aerosol. Oxidizing process gases and/or process aerosols which are employed can include oxygen-containing gases and/or aerosols, such as oxygen (O₂), carbon dioxide (CO₂), carbon monoxide (CO), ozone (O₃), hydrogen peroxide gas (H₂O₂), water vapour (H₂O), or vaporised methanol (CH₃OH); and/or nitrogen-containing gases and/or aerosols, such as nitrous gases (NOₓ), nitrous oxide (N₂O), nitrogen (N₂).
(2) A second chemically oxidizing treatment involves a two step process of exposing the mandrel surface to an inert gas plasma to generate reactive sites followed by quenching with an oxidizing gas, preferably air or oxygen.
(3) A third chemically oxidizing treatment process involves steps of (a) exposing the mandrel surface first to a plasma of a cross-linkable gas; (b) plasma polymerizing a coating derived from the cross-linkable gas onto the mandrel surface; (c) followed by exposure of the surface of the coated mandrel to a chemically oxidizing environment as described in (1) above. Cross-linkable process gasses include include, preferably, unsaturated hydrocarbons, such as ethylene, propylene, butene or acetylene; saturated hydrocarbons with the general composition Cₙ, H₂ₙ₊₂, such as methane, ethane, propane, butane, pentane, iso-propane or iso-butane; vinyl compounds, such as vinyl acetate or methyl vinyl ether; acrylates, such as acrylic acid, methacrylic acid or methyl methacrylate; silanes of the general composition Siₙ, H₂ₙ₊₂, halogenated silicon hydrides, such as SiCl₄, SiCI₃H, SiCI₂H₂, or SiCIH₃, or alkoxysilanes, such as tetraethoxysilane; hexamethyldisilazane; or hexamethyldisiloxane.

Further examples of plasma surface treatment methods are described in U.S. Patent No. 6,613,394 which is incorporated herein by reference in its entirety.

### Solution Oxidation Methods

Solution oxidation methods include treatment with an oxidizing chemical reagent such as hydrogen peroxide, chromic acid, chlorine in water, or a permanganate salt solution to increase the surface energy of the material.

### Flame Oxidation Methods

Flame treatment methods know for use in surface treatment of plastics to increase adhesion properties for bonding or printing can also be used to increase the surface energy of the material.

### Corona Discharge Methods

Corona discharge or electrical discharge in an oxidizing gas, such as air, can also be used to increase the surface energy of the material.

### Light Treatment Methods

Light exposure, preferably UV light exposure, in the presence of an oxidizing gas, preferably air, oxygen or ozone can be used to increase the surface energy of the material.

### Coating Methods

A coating with higher surface energy can be deposited on the silicone rubber surface, such as by plasma polymerization or by chemical vapor deposition (CVD) to increase the surface energy of the surface. Further, a deposited coating may be further treated by a wettability enhancing method described above. The coating can be a conformal coating.

Other coatings can includes a plasma polymerized or CVD coating which forms an impervious coating on the mandrel surface. An impervious coating can prohibit the migration of any agents that could reduce the wettability of the surface from within the body of the silicone rubber to the surface. Specifically, the impervious coating would prohibit the migration and accumulation of silicone oils or low molecular weight silicone species to the silicone rubber or elastomer surface.

### Extracting Methods

Extraction to remove compounds such as oils and waxes which decrease the surface energy of a material can be employed. Extraction can be used alone or in addition to any of the other treatment methods described above. When surface treatment methods are used to increase the surface energy of a material, the effectiveness of this treatment can be decreased if materials, such as oils, from within the material subsequently pass to the surface. Extraction reduces this phenomenon. Extraction processes can include use of solvent, heating, or other processes to remove fugitive compounds from within a material. Fugitive compounds which may be retained within a material and can affect surface energy include silicone oil, other oils, processing aids, waxes, and the like.

In one example of a solvent extraction process, a mandrel covered with silicone rubber is placed into a solvent such as methylene chlorine, heptane, anisole, tolulene, or xylene for a period of time to remove a substantial amount of the silicone oils retained in the material. The solvent causes the silicone rubber material to swell and the silicone oil passes into the solvent. Multiple sequential solvent extracts may be required to reduce the oil to a level that does not deleteriously affect the wettability.

### Metal Treatment Methods

In addition to or as an alternative to treating the surfaces of the mandrel or other temporary bottom surface of the holes, the surfaces of the stent may also be treated. Either the entire surface of the stent or the walls of the holes can be treated to lower the surface energy of the metal or other stent material. The surface energy of a metal can be lowered by treatment with a hydrophobic agent that is complexed or chemically bonded to the surface. Such agents include polyvalent metal salts of fatty acids, such as calcium, magnesium, or zinc stearates or palmitates.

The surface energies of various materials for use in the present invention are listed below. These surface energies can be modified by one or more of the methods described above to achieve desired surface energies for the surfaces of the holes for loading of a beneficial agent.

| Material | Surface energy (dvne/cm) |
|---|---|
| PTFE (Teflon) | 19 |
| Silicone | 20 - 24 |
| Poly (vinylidene fluoride) | 25 |
| Poly (propylene) | 29 |
| Poly (ethylene) | 33 |
| Poly (styrene) | 33 |
| Amylopectin | 35 |
| Poly (vinyl acetate) | 37 |
| Poly (vinyl alcohol) | 37 |
| Poly (vinyl chloride) | 39 |
| Starch | 39 |
| Poly(methyl methacrylate)(PMMA) | 40 |
| Poly (sulfone) | 41 |
| Poly (carbonate) | 42-45 |
| Poly (ethylene terephthalate) | 43 |
| Poly (acrylonitrile) | 44 |
| Cellulose | 44 |
| Nylon | 43-46 |
| Stainless Steel | >100 |
| Cobalt Chrome Alloy | >100 |

The surface tensions of some examples of solvents are listed below. The surface energies of the solutions including these solvents will vary depending on the polymers and/or drugs contained in the solution. However, when the amount of solvent in the solution is high the surface tension of the solvent may be used as an estimate of the solution surface tension.

| Solvent | Surface tension dyne/cm) |
|---|---|
| Silicone oil | 21 |
| Ethanol | 22. |
| Methanol | 22.9 |
| Acetone | 23.8 |
| Toluene | 27 |
| Xylene | 28 |
| Methylene chloride | 28.2 |
| Ethyl lactate (EL) | 30 |
| Anisole | 35 |
| Dimethylformamide (DMF) | 37 |
| Benzyl alcohol | 39 |
| N-Methyl pyrrolidone (NMP) | 40 |
| Dimethyl sulfoxide (DMSO) | 43.8 |
| Water | 72.8 |

The methods and systems for loading a medical device with a beneficial agent are described herein primarily for use with a stent. The invention may also be used with other types of implantable medical devices for the benefit of the patient. Specifically, it is envisioned that the stent is a vascular stent containing beneficial agents for the treatment of restenosis and/or other vascular conditions. For example, the beneficial agents can include one or more antiproliferative, antineoplastic, anti inflammatory, antiplatelet, anticoagulant, antifibrin, antithrombin, antimitotic, antibiotic, or antioxidant substances.

The methods and systems may also be used for loading other drug delivery devices including for example, drug delivery devices for delivery of chemotherapeutic agents, antibiotics, or anti inflammatory agents. The implantable drug delivery devices can be configured in a variety of shapes depending on the location where in the device will be implanted. Some examples of device shapes which may be loaded with a beneficial agent include coils, meshes, filaments, cylinders, discs, and ocular implants.

When implants having shapes other than generally tubular shapes are loaded according to the present invention, the mandrel can be replaced with a temporary bottom for the holes having another shape, such as a cradle shaped temporary bottom for filling holes in a filament.

In one alternative embodiment, an implantable drug delivery device has holes having permanent bottoms, i.e., the holes are wells or recesses. In this case, the entire holes or the entire implantable device can be treated to increase the surface energy of the surface above the surface tension of the liquid solution used for filling. For example a plastic implantable drug delivery device can be treated to increase its surface energy above a surface tension of a liquid filling solution to improved deposition of the beneficial agent in the holes.

The liquid beneficial agent solutions loaded into the holes of the implantable medical device can be provided in liquid form by incorporation of a solvent or a combination of solvents. When a solvent is used, evaporation of the solvent causes solidification of the matrix. Alternatively, the liquid beneficial agent can be polymer only or a combination of polymer and drug without solvent which is loaded in a heated state. Once loaded, the liquid solution is cooled and solidified. A combination of some solvent and heating can also be used to maintain the liquid beneficial agent in a liquid state for loading in the holes.

Polymers are generally used as a carrier for drug loaded in the holes and for non-drug layers including base layers, cap layers, barrier layers, and separating layers. However, other non-polymer matrix materials may also be used either alone or in combination with the polymers. Examples of non-polymer matrix materials include saccarides and carbohydrates. The matrix materials are preferably bioresorbable so that upon delivery of all the drug the holes are empty. The use of bioresorbable materials also allows delivery of all the drug without sequestering of drug within the holes.

In one alternative embodiment, a non bioerodible matrix material base layer or a slowly eroding base layer having a hole, such as the hole shown in FIG. 2, can be used to control luminal release of a drug. In this example, the base layer can be formed with a hole of a desired size by controlling the relative surface tension of the liquid solution and surface energy of the temporary bottom surface. A base layer with a hole in the bottom of a controlled size can retard delivery of drug to the luminal side of the cylindrical device without completely preventing delivery.

### Therapeutic Agents

Therapeutic agents for use with the present invention which may be use alone or in combination may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, micro spheres, micro bubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, tacrolimus, everolimus, and other limus drugs, paclitaxel, actinomycin D, vinca alkaloids, colchicines, restin NG, PPAR gamma agonists, and cyclosporin.

Antithrombins include, without limitation, heparin, plasminogen, α₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation, glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limitation, Adiponectin.

While the invention has been described in detail with reference to the preferred embodiments thereof, it will be apparent to one skilled in the art that various changes and modifications can be made and equivalents employed, without departing from the present invention that is defined by the claims.

## Claims

1. A method of loading an implantable medical device (10) with a beneficial agent, the method comprising:
providing an implantable device body having a plurality of through holes (12);
**characterized by**
providing a sealing member (22) for temporarily sealing a bottom of the plurality of through holes (12), the temporary sealing member (22) having a resilient sealing surface and a surface energy;
treating the temporary sealing member (22) to increase the surface energy of the temporary sealing member (22);
substantially sealing a bottom of the plurality of holes (12) with the temporary sealing member (22); and
loading a fluid beneficial agent into the holes (12), wherein a surface tension of the fluid beneficial agent is less than the surface energy of the temporary sealing member (22) to achieve wetting of the temporary sealing member (22).

2. The method of claim 1, wherein the temporary sealing member (22) is resilient.

3. The method of claim 1, wherein the temporary sealing member (22) is a mandrel (20).

4. The method of claim 3, further comprising a step of treating the mandrel (20) to increase the surface energy of the mandrel surface.

5. The method of claim 1, further comprising a step of treating the temporary sealing member (22) to increase the surface energy of the temporary sealing member (22).

6. The method of claim 5, wherein the step of treating comprises exposing at least a portion of the surface of the temporary sealing member (22) to a chemically oxidizing environment for a time sufficient to increase the surface energy of the temporary sealing member (22) by at least 2 dynes/cm.

7. The method of claim 6, wherein the chemically oxidizing environment comprises a plasma comprising an inert process gas and an oxidizing process gas.

8. The method of claim 6, wherein the step of exposing to a chemically oxidizing environment comprises corona discharge or electrical discharge in an oxidizing gas.

9. The method of claim 5, wherein the step of treating the temporary sealing member (22) comprises at least one of flame oxidation treatment, solution oxidation treatment, corona discharge treatment, light treatment, and coating.

10. The method of claim 5 or 9, wherein a step of extracting the temporary sealing member (22) using a solvent precedes the step of treating.

11. The method of claim 1, wherein the step of treating the temporary sealing member (22) comprises removing compounds which decrease the surface energy of the material by placing the temporary sealing member (22) into a solvent which extracts oils from the temporary sealing member (22), wherein the solvent extraction step is repeated until the surface energy of the temporary sealing member (22) is greater than the surface tension of the fluid beneficial agent.

12. The method of claim 1, wherein the fluid beneficial agent comprises a polymer and a solvent.

13. The method of claim 1, wherein the fluid beneficial agent comprises a polymer in fluid form.

14. The method of claim 1, wherein the fluid beneficial agent comprises a drug.

15. The method of claim 1, wherein the implantable device body is formed of a metal, and the temporary sealing member (22) is formed of a material having a surface energy lower than that of the metal.

16. A system for loading an implantable medical device (10) with a beneficial agent, the system comprising:
an implantable medical device body having a plurality of through holes (12) therein;
**characterized by**
a sealing member (22) for temporarily sealing a bottom of the plurality of through holes (12), the temporary sealing member (22) having a resilient sealing surface and a surface energy, wherein the temporary sealing member (22) is treated to increase the surface energy of the temporary sealing member (22);
bottom surfaces of the plurality of holes (12) being substantially sealed with the temporary sealing member (22);
a dispenser (30) for dispensing a fluid solution of beneficial agent into the holes (12); and
a supply of the fluid solution connected to the dispenser (30), wherein the fluid solution has a surface tension less than the surface energy of the temporary sealing member (22) to achieve wetting of the temporary sealing member (22).

17. The system of claim 16, wherein the temporary sealing member (22) is a mandrel (20).

18. The system of claim 16, wherein the temporary sealing member (22) is resilient.

19. The system of claim 16, wherein the fluid solution comprises a polymer and a solvent.

20. The system of claim 16, wherein the fluid solution comprises a polymer in fluid form.

21. The system of claim 16, wherein the fluid solution comprises a drug.

22. The system of claim 16, wherein the implantable medical device (10) is formed of a metal, and the bottom surfaces of the holes (12) are formed of a resilient material.

## Patentansprüche

1. Verfahren zum Befüllen einer implantierbaren medizinischen Vorrichtung (10) mit einem nützlichen Agens, das Verfahren beinhaltend:
Bereitstellen eines implantierbaren Vorrichtungskörpers mit mehreren Durchgangslöchern (12);
**gekennzeichnet durch**
Bereitstellen eines Dichtglieds (22) zum zeitweisen Abdichten des Bodens der mehreren Durchgangslöcher (12), wobei das zeitweise Dichtglied (22) eine nachgiebige Dichtfläche und eine Oberflächenenergie aufweist;
Behandeln des zeitweisen Dichtglieds (22) zum Erhöhen der Oberflächenenergie des zeitweisen Dichtglieds (22);
im Wesentlichen Abdichten des Bodens der mehreren Löcher (12) mit dem zeitweisen Dichtglied (22); und
Füllen eines flüssigen nützlichen Agens in die Löcher (12), wobei die Oberflächenspannung des flüssigen nützlichen Agens geringer ist als die Oberflächenenergie des zeitweisen Dichtglieds (22), zum Erreichen der Benetzung des zeitweisen Dichtglieds (22).

2. Verfahren nach Anspruch 1, wobei das zeitweise Dichtglied (22) nachgiebig ist.

3. Verfahren nach Anspruch 1, wobei das zeitweise Dichtglied (22) ein Dorn (20) ist.

4. Verfahren nach Anspruch 3, ferner beinhaltend einen Schritt des Behandelns des Dorns (20) zum Erhöhen der Oberflächenenergie der Dorn-Oberfläche.

5. Verfahren nach Anspruch 1, ferner beinhaltend einen Schritt des Behandelns des zeitweisen Dichtglieds (22) zum Erhöhen der Oberflächenenergie des zeitweisen Dichtglieds (22).

6. Verfahren nach Anspruch 5, wobei der Schritt des Behandelns das Aussetzen wenigstens eines Teils der Oberfläche des zeitweisen Dichtglieds (22) einer chemisch oxidierenden Umgebung für eine ausreichende Zeitspanne, um die Oberflächenenergie des zeitweisen Dichtglieds (22) um mindestens 2 dyn/cm zu erhöhen.

7. Verfahren nach Anspruch 6, wobei die chemisch oxidierende Umgebung ein Plasma beinhaltet, welches ein inertes Prozessgas und ein oxidierendes Prozessgas beinhaltet.

8. Verfahren nach Anspruch 6, wobei der Schritt des Aussetzens einer chemisch oxidierenden Umgebung eine Korona-Entladung oder eine elektrische Entladung in einem oxidierenden Gas beinhaltet.

9. Verfahren nach Anspruch 5, wobei der Schritt des Behandelns des zeitweisen Dichtglieds (22) wenigstens eines von Flammen-Oxidations-Behandlung, Lösungs-Oxidations-Behandlung, Korona-Entladungs-Behandlung, Licht-Behandlung und Beschichten beinhaltet.

10. Verfahren nach Anspruch 5 oder 9, wobei ein Schritt des Extrahierens des zeitweisen Dichtglieds (22) unter Verwendung eines Lösungsmittels dem Schritt des Behandelns vorausgeht.

11. Verfahren nach Anspruch 1, wobei der Schritt des Behandelns des zeitweisen Dichtglieds (22) das Entfernen von Verbindungen beinhaltet, die die Oberflächenenergie des Materials erniedrigen , indem das zeitweise Dichtglied (22) in ein Lösungsmittel gegeben wird, welches Öle aus dem zeitweisen Dichtglied (22) extrahiert, wobei der Lösungsmittel-Extraktionsschritt wiederholt wird, bis die Oberflächenenergie des zeitweisen Dichtglieds (22) größer ist als die Oberflächenspannung des flüssigen nützlichen Agens.

12. Verfahren nach Anspruch 1, wobei das flüssige nützliche Agens ein Polymer und ein Lösungsmittel beinhaltet.

13. Verfahren nach Anspruch 1, wobei das flüssige nützliche Agens ein Polymer in flüssiger Form beinhaltet.

14. Verfahren nach Anspruch 1, wobei das flüssige nützliche Agens einen Wirkstoff beinhaltet.

15. Verfahren nach Anspruch 1, wobei der implantierbare Vorrichtungskörper aus einem Metall gebildet ist, und das zeitweise Dichtglied (22) aus einem Material mit niedrigerer Oberflächenenergie als der des Metalls gebildet ist.

16. System zum Befüllen einer implantierbaren medizinischen Vorrichtung (10) mit einem nützlichen Agens, das System beinhaltend:
einen implantierbaren medizinischen Vorrichtungskörper mit mehreren Durchgangslöchern (12) darin;
**gekennzeichnet durch**
ein Dichtglied (22) zum zeitweisen Abdichten eines Bodens der mehreren Durchgangslöcher (12), wobei das zeitweise Dichtglied (22) eine nachgiebige Abdicht-Oberfläche und eine Oberflächenenergie aufweist, wobei das zeitweise Dichtglied (22) behandelt ist, um die Oberflächenenergie des zeitweisen Dichtglieds (22) zu erhöhen;
wobei Bodenöffnungen der mehreren Löcher (12) im Wesentlichen mit dem zeitweisen Dichtglied (22) abgedichtet sind;
einen Verteiler (30) zum Verteilen einer flüssigen Lösung nützlichen Agens' in die Löcher (12); und
eine mit dem Verteiler (3) verbundene Zufuhr der flüssigen Lösung, wobei die flüssige Lösung eine geringere Oberflächenspannung aufweist als die Oberflächenenergie des zeitweisen Dichtglieds (22), um Benetzen des zeitweisen Dichtglieds (22) zu erreichen.

17. System nach Anspruch 16, wobei das zeitweise Dichtglied (22) ein Dorn (20) ist.

18. System nach Anspruch 16, wobei das zeitweise Dichtglied (22) nachgiebig ist.

19. System nach Anspruch 16, wobei die flüssige Lösung ein Polymer und ein Lösungsmittel beinhaltet.

20. System nach Anspruch 16, wobei die flüssige Lösung ein Polymer in flüssiger Form beinhaltet.

21. System nach Anspruch 16, wobei die flüssige Lösung einen Wirkstoff beinhaltet.

22. System nach Anspruch 16, wobei die implantierbare medizinische Vorrichtung (10) aus einem Metall gebildet ist, und die Bodenöffnungen der Löcher (12) aus einem nachgiebigen Material gebildet sind.

## Revendications

1. Procédé de chargement d'un dispositif médical implantable (10) avec un agent bénéfique, le procédé comprenant les étapes suivantes :
prévoir un corps de dispositif implantable ayant une pluralité de trous traversants (12) ;
**caractérisé par** les étapes suivantes :
prévoir un organe d'étanchéité (22) pour rendre temporairement étanche un fond de la pluralité de trous traversants (12), l'organe d'étanchéité temporaire (22) ayant une surface d'étanchéité résiliente et une énergie de surface ;
traiter l'organe d'étanchéité temporaire (22) pour accroître l'énergie de surface de l'organe d'étanchéité temporaire (22) ;
rendre sensiblement étanche un fond de la pluralité de trous (12) avec l'organe d'étanchéité temporaire (22) ; et
charger un agent bénéfique fluide dans les trous (12), dans lequel une tension de surface de l'agent bénéfique fluide est inférieure à l'énergie de surface de l'organe d'étanchéité temporaire (22) pour obtenir une humidification de l'organe d'étanchéité temporaire (22).

2. Procédé de la revendication 1, dans lequel l'organe d'étanchéité temporaire (22) est résilient.

3. Procédé de la revendication 1, dans lequel l'organe d'étanchéité temporaire (22) est un mandrin (20).

4. Procédé de la revendication 3, comprenant également une étape de traitement du mandrin (20) pour accroître l'énergie de surface de la surface de mandrin.

5. Procédé de la revendication 1, comprenant également une étape de traitement de l'organe d'étanchéité temporaire (22) pour accroître l'énergie de surface de l'organe d'étanchéité temporaire (22).

6. Procédé de la revendication 5, dans lequel l'étape de traitement comprend l'exposition d'une partie au moins de la surface de l'organe d'étanchéité temporaire (22) à un environnement chimiquement oxydant pendant une durée suffisante pour accroître d'au moins 2 dynes/cm l'énergie de surface de l'organe d'étanchéité temporaire (22).

7. Procédé de la revendication 6, dans lequel l'environnement chimiquement oxydant comprend un plasma comprenant un gaz de procédé inerte et un gaz de procédé oxydant.

8. Procédé de la revendication 6, dans lequel l'étape d'exposition à un environnement chimiquement oxydant comprend une décharge par effet corona ou décharge électrique dans un gaz oxydant.

9. Procédé de la revendication 5, dans lequel l'étape de traitement de l'organe d'étanchéité temporaire (22) comprend l'un au moins des traitements suivants :
traitement par oxydation à la flamme, traitement par oxydation en solution,
traitement par décharge par effet corona, traitement à la lumière, et enduction.

10. Procédé de la revendication 5 ou 9, dans lequel une étape d'extraction de l'organe d'étanchéité temporaire (22) utilisant un solvant précède l'étape de traitement.

11. Procédé de la revendication 1, dans lequel l'étape de traitement de l'organe d'étanchéité temporaire (22) comprend la suppression de composants qui réduisent l'énergie de surface du matériau, en plaçant l'organe d'étanchéité temporaire (22) dans un solvant qui extrait les huiles de l'organe d'étanchéité temporaire (22), dans lequel l'étape d'extraction de solvant est répétée jusqu'à ce que l'énergie de surface de l'organe d'étanchéité temporaire (22) soit supérieure à la tension de surface de l'agent bénéfique fluide.

12. Procédé de la revendication 1, dans lequel l'agent bénéfique fluide comprend un polymère et un solvant.

13. Procédé de la revendication 1, dans lequel l'agent bénéfique fluide comprend un polymère sous forme fluide.

14. Procédé de la revendication 1, dans lequel l'agent bénéfique fluide comprend un médicament.

15. Procédé de la revendication 1, dans lequel le corps de dispositif implantable se compose d'un métal, et l'organe d'étanchéité temporaire (22) se compose d'un matériau ayant une énergie de surface inférieure à celle du métal.

16. Système de chargement d'un dispositif médical implantable (10) avec un agent bénéfique, le système comprenant :
un corps de dispositif médical implantable ayant une pluralité de trous traversants (12) ;
**caractérisé par** :
un organe d'étanchéité (22) pour rendre temporairement étanche un fond de la pluralité de trous traversants (12), l'organe d'étanchéité temporaire (22) ayant une surface d'étanchéité résiliente et une énergie de surface, dans lequel l'organe d'étanchéité temporaire (22) est traité pour accroître l'énergie de surface de l'organe d'étanchéité temporaire (22) ;
les surfaces du fond de la pluralité de trous (12) étant rendues sensiblement étanches avec l'organe d'étanchéité temporaire (22) ;
un distributeur (30) pour distribuer, une solution fluide d'agent bénéfique dans les trous (12) ; et
une alimentation en solution fluide reliée au distributeur (30), dans lequel la solution fluide a une tension de surface inférieure à l'énergie de surface de l'organe d'étanchéité temporaire (22) pour obtenir une humidification de l'organe d'étanchéité temporaire (22).

17. Système de la revendication 16, dans lequel l'organe d'étanchéité temporaire (22) est un mandrin (20).

18. Système de la revendication 16, dans lequel l'organe d'étanchéité temporaire (22) est résilient.

19. Système de la revendication 16, dans lequel la solution fluide comprend un polymère et un solvant.

20. Système de la revendication 16, dans lequel la solution fluide comprend un polymère sous forme fluide.

21. Système de la revendication 16, dans lequel la solution fluide comprend un médicament.

22. Système de la revendication 16, dans lequel le dispositif médical implantable (10) se compose d'un métal, et les surfaces du fond des trous (12) se composent d'un matériau résilient.
